# EUROPÄISCHE PATENTSCHRIFT

(11) **EP 0 862 896 B1**
(45) Veröffentlichungstag und Bekanntmachung des Hinweises auf die Patenterteilung: **01.06.2005**
(21) Anmeldenummer: 98103839.1
(22) Anmeldetag: 04.03.1998
(51) Int. Cl.: A61B 5/00, A61C 19/04, G01N 21/64

(54) **Vorrichtung zum Erkennen von Karies, Plaque oder bakteriellem Befall an Zähnen**
Device for detecting caries, plaque or bacterial attack of the teeth
Dispositif pour la détection des caries, de la plaque ou de l'attaque bactérienne dentaire

(30) Priorität: 07.03.1997 DE 29704185 U
(43) Veröffentlichungstag der Anmeldung: 09.09.1998
(73) Patentinhaber: Kaltenbach & Voigt GmbH & Co. KG, 88400 Biberach/Riss (DE)
(72) Erfinder: Heckenberger, Hans, 88400 Biberach (DE)
(74) Vertreter: Schmidt-Evers, Jürgen, Dipl.-Ing.

(56) Entgegenhaltungen:
- DE-A- 4 200 741
- US-A- 5 324 200
- US-A- 5 382 163

## Beschreibung

Die Erfindung betrifft eine Vorrichtung zum Erkennen von Karies, Plaque oder bakteriellem Befall an Zähnen.

Eine derartige Vorrichtung ist beispielsweise aus der DE 30 31 249 C2 bekannt. Dabei wird ein zu untersuchender Zahn mit einer nahezu monochromatischen Lichtquelle bestrahlt, wobei an dem bestrahlten Zahn eine Fluoreszenzstrahlung angeregt wird. Die somit an dem Zahn angeregte Fluoreszenzstrahlung wird erfaßt und ausgewertet, wobei in dem Fluoreszenzspektrum deutliche Unterschiede zwischen kariösen und gesunden Zahnbereichen auftreten. So ist im roten Spektralbereich des Fluoreszenzspektrums des Zahnes (ca. 550 bis 650 nm) die Intensität deutlich höher als bei einem gesunden Zahn. Somit kann aufgrund der Erfassung der Fluoreszenzstrahlung des untersuchten Zahnes ein gesunder Zahnbereich eindeutig von einem kariösen Zahnbereich berührungslos unterschieden werden.

Eine ähnliche Vorrichtung zum Erkennen von Karies ist in der DE 42 00 741 A1 beschrieben, wobei in dieser Druckschrift zudem vorgeschlagen wird, die Fluoreszenz des Zahnes durch eine Anregungsstrahlung mit einer Wellenlänge im Bereich 360 bis 580 nm anzuregen und die somit erhaltene Fluoreszenzstrahlung des Zahnes für Wellenlängen ab 620 nm auszufiltern. Durch diese Maßnahmen ist der Abstand zwischen der Wellenlänge der Anregungsstrahlung und der empfangenen Fluoreszenzstrahlung ausreichend groß, so daß die Anregungsstrahlung nicht die Auswertungsergebnisse durch Überlagerung der Fluoreszenzstrahlung verfälschen kann. Die dort beschriebene Vorrichtung umfaßt eine Emissions- und Erfassungseinrichtung zum Bestrahlen des zu untersuchenden Zahnes mit der Anregungsstrahlung und zum Erfassen einer an dem bestrahlten Zahn angeregten Fluoreszenzstrahlung, die eine Emissionsfaser, welche den zu untersuchenden Zahn mit der Anregungsstrahlung bestrahlt, und Detektionsfasern, welche die an dem bestrahlten Zahn angeregte Fluoreszenzstrahlung erfassen, aufweist.

Auch aus der DE-U1-93 17 984 ist eine Vorrichtung zum Erkennen von Karies bekannt, wobei die Anregungsstrahlung nicht kontinuierlich, sondern gepulst während eines Anregungsintervalls erzeugt wird. Die aufgrund der Anregungsstrahlung an dem Zahn hervorgerufene Fluoreszenzstrahlung des untersuchten Zahnes wird während eines gegenüber dem Anregungsintervall zeitverzögerten Auswerteintervalls erfaßt.

Aus obigen Druckschriften ist es lediglich bekannt, einen zu untersuchenden Zahn mit einem einzigen Lichtleiter zu bestrahlen und die von dem bestrahlten Zahn erzeugte Fluoreszenzstrahlung durch einen neben dem Emissionslichtleiter angeordneten Detektionslichtleiter zu erfassen. Dabei kann es jedoch zu einer inhomogenen Bestrahlung des zu untersuchenden Zahnes kommen und aufgrund der Anordnung des Detektionslichtleiters neben dem Emissionslichtleiter wird das Fluoreszenzspektrum nicht genau erfaßt.

Der vorliegenden Erfindung liegt daher die Aufgabe zugrunde, eine Vorrichtung zum Erkennen von Karies, Plaque oder bakteriellem Befall derart auszugestalten, daß ein zu untersuchender Zahn homogen bestrahlt und das angeregte Fluoreszenzspektrum des Zahnes genau erfaßt werden kann. Zudem soll die Handhabbarkeit der gesamten Vorrichtung und deren Anwendungsbereich verbessert werden.

Diese Aufgabe wird erfindungsgemäß durch die in Anspruch 1 beanspruchte Vorrichtung zum Erkennen von Karies, Plaque oder bakteriellem Befall gelöst.

Erfindungsgemäß weist die Emissions- und Erfassungseinrichtung eine Vielzahl von einzelnen Emissionsfasern und Detektionsfasern auf, die abwechselnd zueinander angeordnet sein können, wobei die Anzahl der Emissionsfasern und Detektionsfasern allgemein zwischen 1 und x liegen kann. Insbesondere kann nur eine Emissionsfaser von mehreren Detektionsfasern koaxial umgeben sein. Die Einzelfasern des Faserlichtleiterbündels der Emissions- und Erfassungseinrichtung, die in der Regel durch eine abnehmbare Lichtsonde gebildet wird, sind von einer Ummantelung umgeben, die beispielsweise aus Metall oder Kunststoff gefertigt sein kann. Die Ummantelung schützt die Einzelfasern des Faserlichtleiterbündels vor Beschädigung und Bruch, so daß durch diesen zusätzlichen Schutz die Handhabbarkeit der Emissions- und Erfassungseinrichtung, d.h. der Lichtsonde, verbessert ist.

Die als Emissions- und Erfassungseinrichtung dienende Lichtsonde ist abnehmbar mit einem zahnärztlichen Handstück gekoppelt, mit dessen Hilfe die Lichtsonde zu einem zu untersuchenden Zahn hingerichtet werden kann. Auch das zahnärztliche Handstück weist ein internes Lichtleitersystem auf, das bei aufgesetzter Lichtsonde mit den Lichtleiterfasern der Lichtsonde gekoppelt ist. Insbesondere weist das zahnärztliche Handstück einen zentralen Emissionslichtleiter auf, der von einer Vielzahl von einzelnen Detektionslichtleiterfasem koaxial umgeben ist. Gemäß einer vorteilhaften Ausführungsform ist die einzige Emissionslichtleiterfaser des zahnärztlichen Handstücks von neun Detektionslichtleiterfasern koaxial umgeben, so daß sich bei einem Emissionslichtleiterfaserdurchmesser von 0,5 mm und einem Detektionslichtleiterfaserdurchmesser von 0,25 mm ein nahezu geschlossener Ringquerschnitt mit einem Durchmesser von 1 mm ergibt.

Die Empfindlichkeit für die Erfassung der am bestrahlten Zahn angeregten Fluoreszenzstrahlung hängt wesentlich von der Gestaltung der als Erfassungseinrichtung dienenden Lichtsonde ab. So kann beispielsweise mit einer abgerundeten oder kegelstumpfartigen Lichtsondenspitze eine deutlich höhere Empfindlichkeit erzielt werden.

Die Bestrahlung eines zu untersuchenden Zahnes erfolgt vorteilhafterweise mit einem Anregungslicht, dessen Wellenlänge im Bereich 600-670 nm liegt. Insbesondere beträgt die Wellenlänge des Anregungslichtes 655 nm.

Da die zur Emission des Anregungslichts und Detektion des angeregten Fluoreszenzspektrums dienende Lichtsonde von dem zahnärztlichen Handstück abnehmbar ausgestaltet ist, kann abhängig von dem jeweiligen Anwendungsbereich, d.h. abhängig von dem zu untersuchenden Zahnbereich, die entsprechend passende Lichtsonde aus einem Satz von austauschbaren Lichtsonden ausgewählt und auf das zahnärztliche Handstück aufgesteckt werden.

Die Unteransprüche beschreiben vorteilhafte Ausführungsformen der vorliegenden Erfindung.

Die Erfindung wird nachfolgend unter Bezugnahme auf die beiliegende Zeichnung anhand bevorzugter Ausführungsbeispiele erläutert. Dabei zeigt:
Fig. 1 eine Gesamtansicht der erfindungsgemäßen Vorrichtung zum Erkennen von Karies, Plaque oder bakteriellem Befall,
Fig. 2 ein Blockdiagramm, welches die Funktionsweise der in Fig. 1 gezeigten erfindungsgemäßen Vorrichtung wiedergibt,
Fig. 3 eine Teilquerschnittsansicht des zahnärztlichen Handstücks und der Lichtsonde, die bei der in Fig. 1 gezeigten erfindungsgemäßen Vorrichtung verwendet werden, sowie Querschnittsansichten von drei verschiedenen Lichtsondenformen,
Fig. 4 eine detaillierte Querschnittsansicht der in Fig. 3b dargestellten Lichtsonde, die bei der in Fig. 1 gezeigten erfindungsgemäßen Vorrichtung zum Einsatz kommt,
Fig. 5 eine Schnittansicht entlang der in Fig. 4 gezeigten strichpunktierten Linie, wobei die sich aus Fig. 5 ergebende Lichtleiterfaseranordnung auch der Lichtleiterfaseranordnung in dem in Fig. 3a gezeigten zahnärztlichen Handstück entsprechen kann, und
Fig. 6 eine zu der in Fig. 5 dargestellten Ansicht ähnlich Schnittansicht der Lichtsonde gemäß einem weiteren Ausführungsbeispiel.

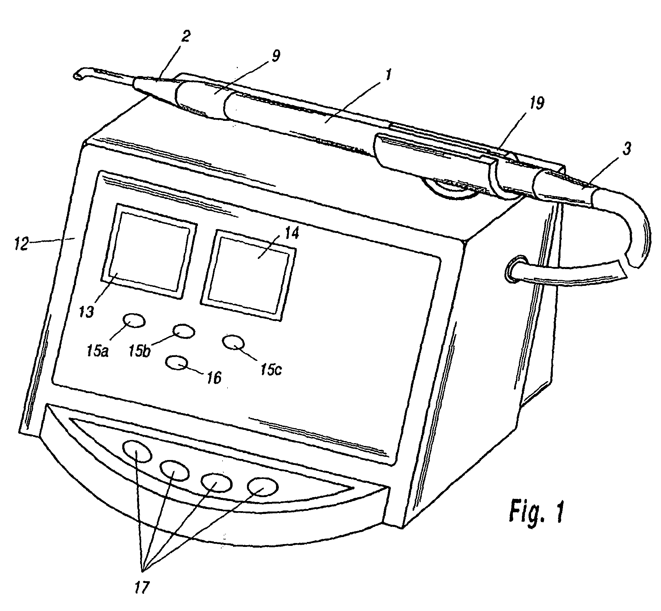

Fig. 1 zeigt eine Gesamtansicht der erfindungsgemäßen Vorrichtung von Karies, Plaque oder bakteriellem Befall.

Die erfindungsgemäße Vorrichtung umfaßt eine Zentraleinheit 12, an die über einen Versorgungsschlauch 3 ein zahnärztliches Handstück 1 mit einer auf das zahnärztliche Handstück 1 aufsteckbaren Lichtsonde 2 angeschlossen ist. Die Zentraleinheit 12 beinhaltet eine Laserlichtquelle, die als Anregungslicht zur Untersuchung eines Zahns dient. Zur Bestrahlung eines zu untersuchenden Zahnes mit Anregungslicht sowie zur Erfassung der von dem zu untersuchenden Zahn kommenden Fluoreszenzstrahlung weist das die Lichtsonde 2 mit der Zentraleinheit 12 verbindende Lichtleitersystem mindestens eine Emissionslichtleiterfaser auf, die das Anregungslicht von der Zentraleinheit 12 zur Lichtsonde 2 leitet, sowie eine Vielzahl von Detektionsfasern, die das Fluoreszenzspektrum des bestrahlten Zahnes erfassen und von der Lichtsonde 2 zu der Zentraleinheit 12 zurückleiten.

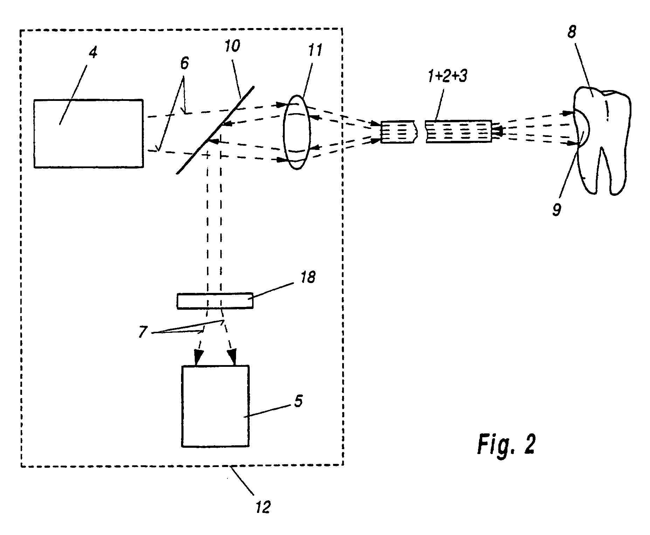

Die Funktionsweise der erfindungsgemäßen Vorrichtung wird dabei aus dem in Fig. 2 gezeigten Blockschaltbild deutlich. Die Zentraleinheit 12 umfaßt eine Lichtquelle 4 zur Erzeugung der Anregungsstrahlung 6 sowie eine Auswertungs- und Erfassungseinrichtung 5 zum Erfassen, Anzeigen sowie gegebenenfalls Auswerten der an dem Zahn 8 angeregten Fluoreszenzstrahlung 7. Die von der Lichtquelle 4 erzeugte Anregungsstrahlung 6 wird über ein Einkopplungslinsensystem 11 dem aus dem Versorgungsschlauch 3, dem zahnärztlichen Handstück 1 und der Lichtsonde 2 bestehenden Lichtleitersystem zugeführt. Die Lichtsonde 2 bestrahlt einen zu untersuchenden Bereich 9 des Zahnes 8 mit der Anregungsstrahlung, deren Wellenlänge vorteilhafterweise im Bereich 600 nm bis 670 nm liegt. Die Detektionsfasern der Lichtsonde 2 erfassen die Fluoreszenzstrahlung des Zahnes 7, die über einen Strahlteiler 10 und ein Spektralfilter 18 der Auswertungs- und Erfassungseinrichtung 5 zugeführt wird. Das Spektralfilter 18 ist dabei vorzugsweise derart ausgestaltet, daß es nur Fluoreszenzstrahlung mit einer Wellenlänge größer als 670 nm durchläßt. Die Auswertungs- und Erfassungseinrichtung 5 wertet die ihr zugeführte Fluoreszenzstrahlung 7 direkt aus und schließt aus der erfaßten Fluoreszenzstrahlung 7 unmittelbar auf das Vorhandensein bzw. Nichtvorhandensein von Karies, Plaque oder bakteriellem Befall. Die Lichtquelle 1 ist vorzugsweise ein HeNe-Laser oder eine Laserdiode. Die Anregungsstrahlung weist insbesondere eine Wellenlänge von ca. 655 nm auf, da bei dieser Wellenlänge der bestmögliche Kompromiß zwischen der verfügbaren Ausgangsleistung, die mit zunehmender Wellenlänge ansteigt und dem spektralen Unterschied zwischen der Anregungsstrahlung und der Fluoreszenzstrahlung, die mit zunehmender Anregungswellenlänge abnimmt, erzielbar ist. Das Spektralfilter 18 besitzt einen Durchlaßbereich ab 670 nm, da bei dem vorgeschlagenen Anregungswellenlängenbereich zwischen 600 nm und 670 nm die Fluoreszenzstrahlung des Zahnes mit Wellenlängen größer als 670 nm den maximalen Abstand zwischen den Fluoreszenzintensitäten für kariöse Bereiche und den Fluoreszenzintensitäten für gesunde Zahnbereiche besitzt.

Die in Fig. 1 gezeigte erfindungsgemäße Vorrichtung weist zudem zwei Anzeigebereiche 13,14 auf, wobei die Anzeige 13 den augenblicklichen Meßwert der Fluoreszenzstrahlung und die Anzeige 14 den während der Messung auftretenden Spitzenwert der Fluoreszenzstrahlung anzeigt. Durch das ständige Anzeigen des Spitzenwerts der Fluoreszenzstrahlung der Anzeige 14 kann der Zahnarzt bei der Behandlung eines Patienten auf einfache Weise durch Vergleich der beiden Anzeigen 13 und 14 wieder die Stelle mit dem größten Kariesbefall auffinden. Die erfindungsgemäße Vorrichtung weist zudem drei Anzeigen 15a-c auf, die durch Aufleuchten die von drei verfügbaren unterschiedlichen Lichtsonden ausgewählte Lichtsonde 2 anzeigen. Um das Gewicht der erfindungsgemäßen Vorrichtung zu verringern und die Mobilität der Vorrichtung zu erhöhen, wird die Vorrichtung vorzugsweise mit einer Batterie oder einem Akkumulator betrieben, wobei eine Warnanzeige 16 durch Aufleuchten das Absinken der von der Batterie bzw. dem Akkumulator gelieferten Versorgungsspannung unter einem Grenzspannungswert anzeigt. In diesem Fall wird der Benutzer durch Aufleuchten der Anzeige 16 aufgefordert, die Batterie auszutauschen oder den Akkumulator erneut aufzuladen. Schließlich weist die in Fig. 1 gezeigte Vorrichtung auch mehrere Tasten 17 zum Kalibrieren der erfindungsgemäßen Vorrichtung vor dessen Inbenutzungsnahme sowie zur Auswahl bestimmter vorgegebener Betriebsmodi auf. Ebenso kann über die Tasten 17 der Typ der auf das zahnärztliche Handstück 1 aufgesteckten Lichtsonde 2 der Zentraleinheit 12 mitgeteilt werden. Insbesondere zum Fixieren des zahnärztlichen Handstücks 1 während des Transports der erfindungsgemäßen Vorrichtung ist eine Ablagehalterung 19 vorgesehen, die drehbar in Stufen oder stufenlos ausgebildet und auch seitlich an der Zentraleinheit 12 angebracht sein kann. An dem zahnärztlichen Handstück 1 ist ein Ringschalter 9 befestigt. Dieser Ringschalter 9 entspricht einem sich in Umfangsrichtung des zahnärztlichen Handstücks 1 erstreckenden ringförmigen Schalter, so daß die Bedienperson unabhängig von der Lage des zahnärztlichen Handstücks 1 in dessen Hand durch Druck mit lediglich einem Finger die erfindungsgemäße Vorrichtung ein- bzw. ausschalten kann. Ebenso kann über den Ringschalter 9 durch kurzes Drücken der durch die Anzeige 14 angezeigte Spitzenwert gelöscht und durch langes Drücken ein neuer Referenzwert gesetzt, d.h. die Lichtsonde 2 abgeglichen werden.

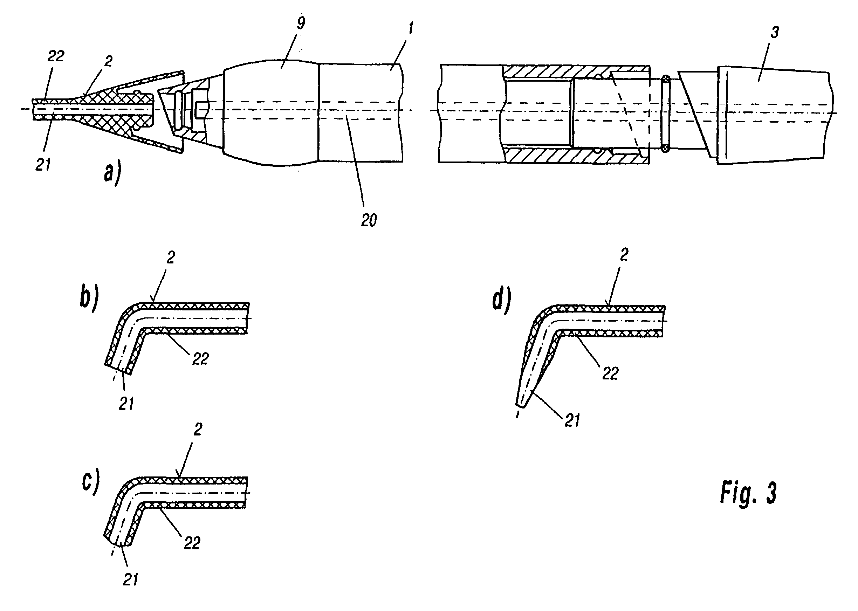

Fig. 3 zeigt eine Teilquerschnittsansicht des Versorgungsschlauches 3 mit dem zahnärztlichen Handstück 1 und einer auf das zahnärztliche Handstück 1 aufsteckbaren Lichtsonde 2 der in Fig. 1 gezeigten Vorrichtung zum Erkennen von Karies. Sowohl die Lichtsonde 2 als auch der Schlauchanschluß des Versorgungsschlauches 3 sind auf das zahnärztliche Handstück 1 aufsteckbar. Das Handstück 1 weist einen internen Kanal 20 auf, in dem das den Schlauch 3 und die Lichtsonde 2 verbindende Lichtleitersystem gelagert ist. Die Lichtsonde 2 weist einen in einer starren Hülle 22 angeordneten Kanal 21 auf, in dem die Emissions- und Detektionsfasern verlaufen. Erfindungsgemäß kann abhängig von der Gestaltung des zu untersuchenden Zahnbereiches aus einer Vielzahl von vorgegebenen Lichtsondenarten ausgewählt werden. So zeigt Fig. 3b den Querschnitt einer Lichtsondenspitze zur Durchsuchung von glatten Zahnoberflächen, Fig. 3c den Querschnitt einer Lichtsondenspitze zur Untersuchung von Fissuren, wobei das zahnseitige Ende dieser Lichtleitersonde abgerundet bzw. kegelstumpfartig ist und Fig. 3d eine Lichtleitersonde zur Untersuchung von Approximalbereichen (Zwischenraumbereichen) eines Zahnes, wobei das Ende dieser Lichtleitersonde spitz verläuft.

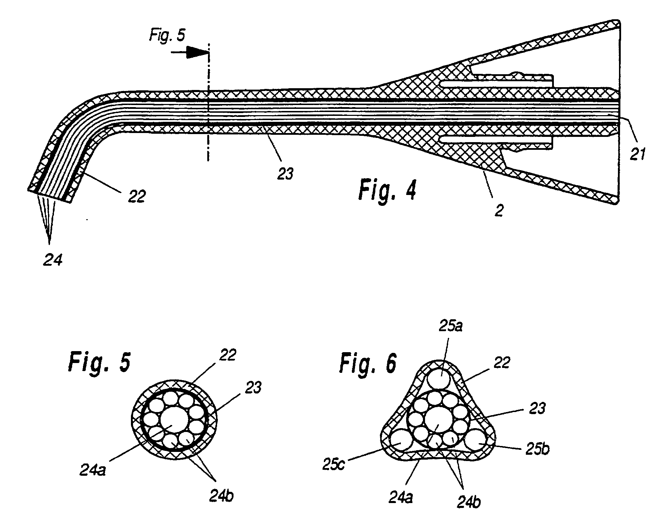

Fig. 4 zeigt eine Querschnittsansicht, durch die in Fig. 3b gezeigte Lichtleitersonde mit darin geführten Lichtleiterfasern. Wie aus Fig. 4 ersichtlich ist, wird eine Vielzahl von einzelnen Lichtleiterfasern 24 in dem Lichtleiterkanal 21 der Lichtleitersonde 2 geführt. Von den einzelnen Lichtleiterfasern 24 dienen einige zur Übertragung des Anregungslichts und andere zur Detektion der von dem zu untersuchenden Zahn abgestrahlten Fluoreszenzstrahlung. Die Anordnung der Anregungslichtleiterfasern gegenüber den Detektionslichtleiterfasern in der Lichtleitersonde 2 ist dabei zunächst nicht festgelegt. Um jedoch eine möglichst homogene Bestrahlung des zu untersuchenden Zahnes sowie eine möglichst gleichmäßige und genauer Erfassung der angeregten Fluoreszenzstrahlung erzielen zu können, ist es vorteilhaft, die Anregungslichtleiterfasern und Detektionslichtleiterfasern abwechselnd zueinander anzuordnen. Insbesondere können die einzelnen Lichtleiterfasern in dem Lichtleiterkanal 21 wie in Fig. 5 gezeigt angeordnet sein, wobei mehrere Detektionslichtleiterfasern 24b konzentrisch um eine Emissionslichtleiterfaser 24a angeordnet sind. Auf diese Weise kann die Detektionssicherheit und -genauigkeit erhöht bzw. stabilisiert werden. Gemäß Fig. 4 und 5 sind die Lichtleiterfasern in einer Hülle 22 angeordnet, die beispielsweise aus Metall oder Kunststoff oder jedem anderen geeigneten Werkstoff gefertigt sein kann. Zudem sind die Lichtleiterfasern auch von einer Ummantelung 23 umgeben. Durch diese Ummantelung wird die Gefahr von Beschädigungen des in dem Lichtleiterkanal 21 geführten Faserbündels verringert und der Bruch der einzelnen Lichtleiterfasern verhindert

Wie bereits anhand Fig. 3 erläutert, weist auch der in Fig. 3 gezeigte interne Kanal 20 des zahnärztlichen Handstücks 1 eine Vielzahl von einzelnen Lichtleiterfasern auf, die entweder das Anregungslicht zur Lichtleitersonde leiten bzw. die von der Lichtleitersonde 2 erfaßte Fluoreszenzstrahlung zu der Auswertungs- und Ermittlungseinrichtung zurückleiten. Dabei werden nach Aufsetzen der Lichtleitersonde 2 auf das zahnärztliche Handstück 1 die in dem Lichtleiterkanal 21 der Lichtleitersonde 2 geführten Lichtleiterfasern mit den in dem Kanal 20 des zahnärztlichen Handstücks 1 geführten Lichtleiterfasern verbunden. Insbesondere kann das in dem Lichtleiterkanal 20 des zahnärztlichen Handstücks 1 geführten Lichtleitersystem die in Fig. 5 gezeigte Anordnung von einzelnen Lichtleiterfasern aufweisen, d.h. in dem Lichtleiterkanal 20 ist lediglich eine zentrale Emissionslichtleiterfaser 24a zur Übertragung des Anregungslichtes vorhanden, wobei eine Vielzahl von einzelnen Detektionslichtleiterfasern 24b koaxial um die Emissionslichtleiterfaser 24a herum angeordnet sind. Werden beispielsweise - wie in Fig. 5 gezeigt - neun Detektionslichtleiterfasern mit einem Faserdurchmesser von 0,25 mm um eine Emissionslichtleiterfaser mit einem Durchmesser von 0,5 mm koaxial angeordnet, ergibt sich ein nahezu geschlossener Ringquerschnitt mit einem Durchmesser von 1 mm.

Es wird jedoch abschließend darauf hingewiesen, daß die Anordnung der Lichtleiterfasern in der Lichtleitersonde 2 nicht notwendigerweise der Anordnung der Lichtleiterfasern in dem zahnärztlichen Handstück entsprechen muß. Vielmehr ist es lediglich erforderlich, daß nach Aufsetzen der Lichtleitersonde 2 auf das zahnärztliche Handstück 1 die in der Lichtleitersonde 2 geführten Emissionslichtleiterfasern bzw. Detektionslichtleiterfasern mit den entsprechenden Emissions- bzw. Detektionslichtleiterfasern des zahnärztlichen Handstücks 1 durch einen entsprechenden Kopplungsmechanismus verbunden werden. So kann beispielsweise das zahnärztliche Handstück die in Fig. 5 gezeigte Lichtleiteranordnung aufweisen, während die Lichtleitersonde 2 nicht nur eine, sondern mehrere Emissionslichtleiterfasern aufweist, die abwechselnd mit den entsprechenden Detektionslichtleiterfasern angeordnet sind, wobei diese mehrere Emissionslichtleiterfasern mit der in Fig. 5 gezeigten zentralen Emissionslichtleiterfaser 24a des zahnärztlichen Handstücks gekoppelt werden. Daher ist es auch möglich, daß der Durchmesser des Faserlichtleiterbündels der Lichtleitersonde größer als der entsprechende Faserlichtleiterbündeldurchmesser des zahnärztlichen Handstücks ist. Insbesondere kann der Durchmesser des Faserlichtleiterbündels der Lichtleitersonde größer als 1 mm sein.

Fig. 6 zeigt eine weitere Schnittansicht einer bei der erfindungsgemäßen Vorrichtung verwendeten Lichtsonde. In diesem Fall sind die einzelnen Lichtleiterfasern 24a, 24b von drei gleichmäßig in Umfangsrichtung der Lichtsonde verteilten Rohren 25a-25c umgeben, die aus festem Material, beispielsweise Metall, gefertigt sind und somit zur Stabilisierung der Lichtsonde dienen. Die Rohre 25a-25c können beispielsweise von einem flexiblen Schrumpfschlauch 22 o.ä. umgeben sein, da bereits durch die Rohranordnung eine ausreichende Stabilität und ein ausreichender Schutz der Lichtleiterfasern gewährleistet ist. Des weiteren können die Rohre 25a-25c auch innerhalb der in Fig. 5 dargestellten festen Ummantelung 22 der Lichtsonde angeordnet sein. Vorteilhafterweise dienen die Rohre nicht nur als Stabilitätsmittel für die Lichtsonde, sondern auch zur Zuführung von Betriebsmitteln, welche für den Betrieb der erfindungsgemäßen Vorrichtung bzw. der Lichtsonde erforderlich bzw. geeignet sind, wie beispielsweise Beleuchtungslicht oder Sprayluft. Insbesondere die Zuführung von Spray- bzw. Blasluft über die Rohre 25a-25c ist hilfreich, da sich herausgestellt hat, daß ein zuverlässigeres Meßergebnis erzielt werden kann, wenn die zu untersuchende Stelle des Zahnes sauber- oder trockengeblasen ist.

## Patentansprüche

1. Vorrichtung zum Erkennen von Karies, Plaque oder bakteriellem Befall an Zähnen, mit einer Einrichtung (4) zum Erzeugen einer Anregungsstrahlung (6), mit einem zahnärztlichen Handstück (1), mit dessen Hilfe die Anregungsstrahlung auf einen zu untersuchenden Zahn (8) zu richten ist, und
mit einem Satz von unterschiedlich geformten austauschbaren Emissions- und Erfassungseinrichtungen (2) zum Bestrahlen des zu untersuchenden Zahnes (8) mit der Anregungsstrahlung (6) und zum Erfassen einer an dem bestrahlten Zahn (8) angeregten Fluoreszenzstrahlung (7), wobei die einzelnen austauschbaren Emissions- und Erfassungseinrichtungen (2) abhängig von der Oberflächengestaltung des zu untersuchenden Zahnbereichs auszuwählen und jeweils mit dem zahnärztlichen Handstück (1) abnehmbar koppelbar sind,
wobei jede Emissions- und Erfassungseinrichtung (2) eine Vielzahl von einzelnen Emissionsfasern (24a), welche den zu untersuchenden Zahn (8) mit der Anregungsstrahlung (6) bestrahlen, und Detektionsfasern (24b), welche die an dem bestrahlten Zahn (8) angeregte Fluoreszenzstrahlung (7) erfassen, aufweist.

2. Vorrichtung nach Anspruch 1,
**dadurch gekennzeichnet,**
**daß** die einzelnen Fasern (24,24a,24b) jeder Emissions- und Erfassungseinrichtung (2) von einer Ummantelung (22) umgeben sind.

3. Vorrichtung nach Anspruch 2,
**dadurch gekennzeichnet,**
**daß** die Ummantelung (22) aus Metall oder Kunststoff gefertigt ist.

4. Vorrichtung nach einem der vorhergehenden Ansprüche,
**dadurch gekennzeichnet,**
**daß** jede Emissions- und Erfassungseinrichtung (2) mindestens ein in ihre Längsrichtung verlaufendes Rohr (25a, 25b, 25c) aufweist.

5. Vorrichtung nach Anspruch 4,
**dadurch gekennzeichnet,**
**daß** jede Emissions- und Erfassungseinrichtung (2) mehrere, im wesentlichen gleichmäßig entlang ihrer Umfangsrichtung verteilte und in ihre Längsrichtung verlaufende Rohre (25a, 25b, 25c) aufweist.

6. Vorrichtung nach Anspruch 4 oder 5,
**dadurch gekennzeichnet,**
**daß** mindestens ein Rohr (25a, 25b, 25c) zur Zuführung eines Betriebsmittels für den Betrieb der Vorrichtung, insbesondere für die Zuführung von Blasluft, vorgesehen ist.

7. Vorrichtung nach einem der vorhergehenden Ansprüche,
**dadurch gekennzeichnet,**
**daß** das zahnärztliche Handstück (1) eine Emissionsfaser (24a) zum Zuführen des Anregungslichts (6) und eine Vielzahl von konzentrisch um die Emissionsfaser (24a) herum angeordneten Detektionsfasern (24b) zum Ableiten der an dem bestrahlten Zahn (8) angeregten Fluoreszenzstrahlung (7) aufweist, wobei nach Kopplung einer Emissions- und Erfassungseinrichtung (2) mit dem zahnärztlichen Handstück (1) die Emissions- und Detektionsfasern der jeweiligen Emissions- und Erfassungseinrichtung (2) mit der Emissions- bzw. den Detektionsfasern des zahnärztlichen Handstücks (1) verbunden sind.

8. Vorrichtung nach Anspruch 7,
**dadurch gekennzeichnet,**
**daß** das zahnärztliche Handstück (1) neun konzentrisch um die Emissionsfaser (24a) herum angeordnete Detektionsfasern (24b) mit einem Durchmesser von jeweils 0,25 mm aufweist, wobei die Emissionsfaser (24a) des zahnärztlichen Handstücks (1) einen Durchmesser von 0,5 mm aufweist.

9. Vorrichtung nach einem der vorhergehenden Ansprüche,
**dadurch gekennzeichnet,**
**daß** jede Emissions- und Erfassungseinrichtung (2) eine Emissionsfaser (24a) und eine Vielzahl von konzentrisch um die Emissionsfaser (24a) herum angeordneten Detektionsfasern (24b) aufweist.

10. Vorrichtung nach einem der vorhergehenden Ansprüche,
**dadurch gekennzeichnet,**
**daß** der Durchmesser des aus den einzelnen Emissions- bzw. Detektionsfasern bestehenden Faserbündels jeder Emissions- und Erfassungseinrichtung (2) größer als 1 mm ist.

11. Vorrichtung nach einem der vorhergehenden Ansprüche,
**dadurch gekennzeichnet,**
**daß** die Wellenlänge des Anregungslichts im Bereich zwischen 600 und 670 nm liegt und insbesondere 655 nm beträgt.

12. Vorrichtung nach einem der vorhergehenden Ansprüche,
**dadurch gekennzeichnet,**
**daß** der Satz von austauschbaren Emissions- und Erfassungseinrichtungen (2) eine Emissions- und Erfassungseinrichtung zum Untersuchen von glatten Zahnoberflächen, eine Emissions- und Erfassungseinrichtung zum Untersuchen von Fissuren und eine Emissionsund Erfassungseinrichtung zum Untersuchen von Zahnzwischenräumen umfaßt.

## Claims

1. Device for the recognition of caries, plaque or bacterial infection of teeth,
having a means (4) for the generation of an excitation radiation (6), having a dental handpiece (1) with the aid of which the excitation radiation can be directed at a tooth (8) to be investigated, and
having a set of differently formed exchangeable emission and acquisition devices (2) for the irradiation of the tooth to be investigated with the excitation radiation (6) and for acquiring a fluorescence radiation (7) excited at the irradiated tooth (8), whereby the individual exchangeable emission and acquisition devices (2) are to be selected in dependence upon the surface property of the tooth region to be investigated and can each be removably coupled with the dental handpiece (1),
whereby each emission and acquisition device (2) has a plurality of individual emission fibers (24a) which irradiate the tooth (8) to be investigated with the excitation radiation (6), and detection fibers (24b) which acquire the fluorescence radiation (7) excited at the irradiated tooth (8).

2. Device according to claim 1,
**characterised in that**,
the individual fibers (24, 24a, 24b) of each emission and acquisition device (2) are surrounded by a sheathing (22).

3. Device according to claim 2,
**characterised in that**,
the sheathing (22) is manufactured of metal or plastics.

4. Device according to any preceding claim,
**characterised in that**,
each emission and acquisition device (2) has at least one tube (25a, 25b, 25c) running in its longitudinal direction.

5. Device according to claim 4,
**characterised in that**,
each emission and acquisition device (2) has a plurality of tubes (25a, 25b, 25c), substantially uniformly distributed along its circumferential direction and running in its longitudinal direction.

6. Device according to claim 4 or 5,
**characterised in that**,
there is provided at least one tube (25a, 25b, 25c) for the delivery of an operating medium for the operation of the device, in particular for the delivery of blasting air.

7. Device according to any preceding claim,
**characterised in that**,
the dental handpiece (1) has an emission fibre (24a) for the delivery of excitation light (6) and a plurality of detection fibers (24b) arranged concentrically around the emission fiber (24a) for the taking off of the fluorescence radiation (7) excited at the irradiated tooth (8), whereby after coupling of an emission and acquisition device (2) with the dental handpiece (1) the emission and detection fibers of the respective emission and acquisition device (2) are coupled with the emission and detection fibres of the dental handpiece (1).

8. Device according to claim 7,
**characterised in that**,
the dental handpiece (1) has nine detection fibers (24b), each of a diameter of 0.25 mm, arranged concentrically around the emission fibre (24a), the emission fibre (24a) of the dental handpiece (1) having a diameter of 0.5 mm.

9. Device according to any preceding claim,
**characterised in that**,
each emission and acquisition device (2) has an emission fibre (24a) and a plurality of detection fibers (24b) arranged concentrically around the emission fibre (24a).

10. Device according to any preceding claim,
**characterised in that**,
the diameter of the fibre bundle, consisting of the individual emission and detection fibers, of each emission. and acquisition device (2) is greater than 1 mm.

11. Device according to any preceding claim,
**characterised in that**,
the wavelength of the excitation light is in the range between 600 and 670 nm, and in particular is 655 nm.

12. Device according to any preceding claim,
**characterised in that**,
the set of exchangeable emission and acquisition devices (2) includes an emission and acquisition device for the investigation of smooth tooth surfaces, an emission and acquisition device for the investigation of fissures, and an emission and acquisition device for the investigation of spaces between teeth.

## Revendications

1. Dispositif permettant de détecter la présence de caries, de plaque dentaire ou d'atteinte bactérienne sur les dents, comportant un dispositif (4) permettant la production d'un rayonnement d'excitation (6), une pièce à main dentaire (1) à l'aide de laquelle le rayonnement d'excitation doit être dirigé sur la dent à examiner (8) et comportant un jeu de dispositifs d'émission et de détection (2) de différentes formes et interchangeables permettant d'irradier la dent à examiner (8) par le rayonnement d'excitation (6) et de détecter le rayonnement de fluorescence (7) excité sur la dent irradiée (8), les différents dispositifs d'émission et de détection (2) interchangeables devant être choisis en fonction de l'état de surface de la zone de dent à examiner et pouvant être couplés de manière amovible à la pièce à main dentaire (1),
chaque dispositif d'émission et de détection (2) comportant un grand nombre de fibres d'émission différentes (24a), lesquelles irradient la dent à examiner (8) avec le rayonnement d'excitation (6) et de fibres de détection (24b) qui détectent le rayonnement de fluorescence (7) excité sur la dent irradiée (8).

2. Dispositif selon la revendication 1,
**caractérisé en ce que**
les différentes fibres (24, 24a, 24b) de chaque dispositif d'émission et de détection (2) sont entourées d'une gaine (22).

3. Dispositif selon la revendication 2,
**caractérisé en ce que**
la gaine (22) est en métal ou en matière plastique.

4. Dispositif selon l'une quelconque des revendications précédentes,
**caractérisé en ce que**
chaque dispositif d'émission et de détection (2) présente au moins un tube passant dans son sens longitudinal (25a, 25b, 25c).

5. Dispositif selon la revendication 4,
**caractérisé en ce que**
chaque dispositif d'émission et de détection (2) présente plusieurs tubes (25a, 25, 25c) répartis essentiellement de manière homogène le long de son sens périphérique et passant dans son sens longitudinal.

6. Dispositif selon la revendication 4 ou 5,
**caractérisé en ce que**
au moins un tube (25a, 25b, 25c) est prévu pour transporter un agent permettant le fonctionnement du dispositif, en particulier pour transporter de l'air de soufflage.

7. Dispositif selon l'une quelconque des revendications précédentes,
**caractérisé en ce que**
la pièce à main dentaire (1) présente une fibre d'émission (24a) permettant de transporter la lumière d'excitation (6) et un grand nombre de fibres de détection (24b), disposées concentriquement autour de la fibre d'émission (24a), permettant de dévier le rayonnement de fluorescence (7) excité sur la dent irradiée (8), tandis qu'après le couplage d'un dispositif d'émission et de détection (2) avec la pièce à main dentaire (1), les fibres d'émission et de détection du dispositif d'émission et de détection (2) concerné sont reliées aux fibres d'émission respectivement de détection de la pièce à main dentaire (1).

8. Dispositif selon la revendication 7,
**caractérisé en ce que**
la pièce à main dentaire (1) présente neuf fibres de détection (24b), disposées concentriquement autour de la fibre d'émission (24a), ayant chacune un diamètre de 0,25 mm, les fibres d'émission (24a) de la pièce à main dentaire (1) présentant un diamètre de 0,5 mm.

9. Dispositif selon l'une quelconque des revendications précédentes,
**caractérisé en ce que**
chaque dispositif d'émission et de détection (2) présente une fibre d'émission (24a) et un grand nombre de fibres de détection (24b) disposées concentriquement autour de la fibre d'émission (24a).

10. Dispositif selon l'une quelconque des revendications précédentes,
**caractérisé en ce que**
le diamètre du faisceau de fibres, constitué des différentes fibres d'émission respectivement de détection, de chaque dispositif d'émission et de détection (2) est supérieur à 1 mm.

11. Dispositif selon l'une quelconque des revendications précédentes,
**caractérisé en ce que**
la longueur d'onde de la lumière d'excitation est comprise entre 600 et 670 mm et, en particulier, de l'ordre de 655 nm.

12. Dispositif selon l'une quelconque des revendications précédentes,
**caractérisé en ce que**
le jeu de dispositifs d'émission et de détection interchangeables (2) comporte un dispositif d'émission et de détection permettant d'examiner des surfaces de dent lisses, un dispositif d'émission et de détection permettant d'examiner des fissures et un dispositif d'émission et de détection permettant d'examiner des espaces interdentaires.
